# EUROPÄISCHE PATENTSCHRIFT

(11) **EP 1 248 811 B1**
(45) Veröffentlichungstag und Bekanntmachung des Hinweises auf die Patenterteilung: **03.08.2005**
(21) Anmeldenummer: 01942269.0
(22) Anmeldetag: 05.01.2001
(51) Int. Cl.: C08G 18/79

(54) **CARBODIIMIDE MIT CARBOXYL- ODER CARBOXYLATGRUPPEN**
CARBODIIMIDES WITH CARBOXYL OR CARBOXYLATE GROUPS
CARBODIIMIDE A GROUPES CARBOXYLE OU CARBOXYLATE

(30) Priorität: 11.01.2000 DE 10000656
(43) Veröffentlichungstag der Anmeldung: 16.10.2002
(73) Patentinhaber: BASF AKTIENGESELLSCHAFT, 67056 Ludwigshafen (DE)
(72) Erfinder: HÄBERLE, Karl, 67346 Speyer (DE); LICHT, Ulrike, 68309 Mannheim (DE); KIELHORN-BAYER, Sabine, 67133 Maxdorf (DE); LACH, Christian, 67098 Bad Dürkheim (DE); MEYER-ROSCHER, Bernd, 67434 Neustadt (DE); PORZIO, Robert, Shane, Charlotte, NC 28202 (US); STALLER, Christelle, F-67470 Seltz (FR); WEYLAND, Peter, 67227 Frankenthal (DE)
(86) Internationale Anmeldenummer: PCT/EP2001/000057
(87) Internationale Veröffentlichungsnummer: WO 2001/051535

(56) Entgegenhaltungen:
- EP-A- 0 198 343
- EP-A- 0 686 626
- EP-A- 0 952 146
- EP-A- 0 962 490
- US-A- 5 866 715

## Beschreibung

Die Erfindung betrifft Verbindungen mit Carbodiimideinheiten und Carboxyl- oder Carboxylatgruppen (Verbindungen V), abgeleitet von
a) aliphatischen oder araliphatischen C₄- bis C₂₀-Polyisocyanaten (Komponente a)
b) Hydroxycarbonsäuren oder Hydroxycarbonsäuresalzen (Komponente b) und
c) gegebenenfalls weiteren Verbindungen, die Gruppen tragen, die mit Isocyanatgruppen in einer Additionsreaktion reagieren können (Komponente c)
d) gegebenenfalls sonstigen Isocyanaten (Komponente d)
wobei sich die Carbodiimideinheiten im wesentlichen ausschließlich von den Isocyanatgruppen der Komponente (a) ableiten.

Organische Carbodiimide und deren Verwendung als Zusatzmittel zu wässrigen Polymerdispersionen sind bekannt. Sie werden z.B. Polymerdispersionen zugesetzt, um das Molekulargewicht der Polymere zu erhöhen. Um die Carbodiimide auf einfache Weise homogen in der Dispersion verteilen zu können, werden sie mit hydrophilen Gruppen versehen.

Die EP-A-198 343 beschreibt Carbodiimide, die Sulfonatgruppen und gegebenfalls zusätzlich Polyethylenoxideinheiten tragen.

Aus der EP-A-686 626 sind weiterhin Carbodiimide bekannt, bei denen die Hydrophilie durch Ammoniumgruppen, die über Dialkylaminoalkohole eingeführt werden, durch Sulfonatgruppen, die über Salze von hydroxy-funktionellen Alkylsulfonsäuren eingeführt werden oder Polyethylenoxidreste bewirkt wird.

Die vorgenannten Produkte weisen jedoch folgende Nachteile auf:
Kationische Produkte wie durch Ammoniumgruppen hydrophilisierte Carbodiimide sind mit den üblicherweise verwendeten anionisch stabilisierten Dispersionen nicht verträglich.

Die mit Sulfonatgruppen hydrophilisierten Carbodiimide sind schwierig herstellbar. Aufgrund des sehr lipophoben Charakters der als Ausgangsverbindungen eingesetzten Salze gestaltet sich die Umsetzung mit den hydrophoben Isocyanatgruppen-aufweisenden Vorprodukten ausgesprochen schwierig, da die gegenseitige Löslichkeit sehr niedrig ist.

Die Dispersionen, die mit Polyalkylenoxidresten hydrophilisierten Carbodiimide ausgehärtet werden, besitzen eine unerwünschte permanente Hydrophilie.

Aus der DE-A-19821668 sind Carbodiimide auf der Basis von 1,3-Bis-(1-methyl-1-isocyanato-ethyl)benzol, bekannt, bei denen die Hydrophilisierung mit Aminosulfonsäuren bewirkt wird.

Aus der nicht vorveröffentlichten DE-A-19954006 sind Carbodiimide auf der Basis von aliphatischen oder aromatischen Polyisocyanaten bekannt, bei denen die Hydrophilisierung mit Aminocarbonsäuren bewirkt wird.

Die Aufgabe der Erfindung bestand in der Bereitstellung von Carbodiimiden, die mit den üblichen, anionisch stabilisierten Polymerdispersionen verträglich sind, einfach herzustellen sind und den damit gehärteten Dispersionsfilmen keine zusätzliche permanente Hydrophilie verleihen.

Die Verbindungen (V) enthalten bevorzugt 200 bis 2000 mmol/kg, besonders bevorzugt 500 bis 1800 mmol/kg Carboxyl- oder Carboxylatgruppen, bezogen auf das Gewicht der Carbodiimide.

Der Anteil an Carbodiimid-Gruppen beträgt im allgemeinen 0,05 bis 8, bevorzugt 0,10 bis 5 mol/kg bezogen auf das Gewicht der Carbodiimide.

Die Carbodiimideinheiten in den erfindungsgemäßen Carbodiimiden werden im wesentlichen in der Weise gebildet, daß je 2 NCO-Gruppen der Komponente (a) unter Abspaltung von Kohlendioxid zu einer Carbodiimideinheit zusammentreten.

Die Verbindungen (V) enthalten bevorzugt mindestens eine Carbodiimideinheit, bevorzugt mehr als eine Carbodiimideinheiten, besonders bevorzugt beträgt der mittlere Kondensationsgrad (Zahlenmittelwert), d.h. die mittlere Anzahl an Carbodiimideinheiten in den erfindungsgemäßen Carbodiimiden 1 bis 20, insbesondere 2 bis 15.

Als Monomere (a) kommen die üblicherweise in der Polyurethanchemie eingesetzten aliphatischen oder araliphatischen Isocyanate mit 4 bis 20 C-Atomen in Betracht.

Insbesondere zu nennen sind Diisocyanate X(NCO)₂, wobei X für einen aliphatischen Kohlenwasserstoffrest mit 4 bis 12 Kohlenstoffatomen, einen cycloaliphatischen mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-5-isocyanatomethylcyclohexan (IPDI), 2,2-Bis-(4-isocyanatocyclohexyl)-propan, Trimethylhexandiisocyanat, 1,4-Diisocyanatobenzol, 1,3-Bis-(1-methyl-1-isocyanatoethyl)-benzol (TMXDI), die Isomeren des Bis-(4-isocyanatocyclohexyl)methans (HMDI) wie das trans/trans-, das cis/cis- und das cis/trans-Isomere sowie aus diesen Verbindungen bestehende Gemische.

Die erfindungsgemäßen Carbodiimide enthalten also bevorzugt Einheiten der allgemeinen Formel I

-X-N=C=N-X-[-N=C=N-X-]ₙ- I

in der
- X: die oben angegebene Bedeutung hat und
- n: eine ganze Zahl von 0 bis 10, bevorzugt 0 bis 5 bedeutet.

Besonders bevorzugt ist X von TMXDI oder Hexamethylendiisocyanat abgeleitet.

Geeignete Hydroxycarbonsäuren sind z.B. die in Beyer, Lehrbuch der Organischen Chemie, 19. Auflage auf S. 262 ff genannten.

Obwohl auch Säuren mit aromatisch gebundener Hydroxylgruppe geeignet sind, werden Säuren mit aliphatisch gebundener Hydroxylgruppe bevorzugt. Besonders bevorzugt sind Hydroxycarbonsäuren mit beta-ständiger Hydroxylgruppe wie beta-Hydroxypropionsäure oder insbesondere bevorzugt Hydroxypivalinsäure oder alpha, alpha-Hydroxymethylalkansäuren wie z.B. Dimethylolpropionsäure.

Sofern die Hydroxycarbonsäuren in Form ihrer Salze eingesetzt werden, so kommen insbesondere Alkali-, Erdalkali- oder Ammoniumsalze in Betracht.

Bevorzugte Verbindungen (V) sind solche der allgemeinen Formel II

R¹-CO-NH-X-N=C=N-X-[-N=C=N-X-]ₙ-NH-CO-R² II

in der n und X die gleiche Bedeutung wie in der allgemeinen Formel I haben und R¹ und R² von Komponente (b) abgeleitete Reste sind, die sich durch Abstraktion eines an eine Hydroxygruppe gebundenen Wasserstoffatoms von Komponente (b) ableiten.

Die Verbindungen (V) können gegebenenfalls außer den durch die Komponenten (a) und (b) abgeleiteten Struktureinheiten noch weitere Struktureinheiten enthalten, die von den Komponenten (c) und (d) abgeleitet sind und hauptsächlich Urethan- oder Harnstoffeinheiten enthalten. Diese werden gebildet durch Reaktion der Isocyanatgruppen der Komponente (d) mit den gegenüber Isocyanat reaktiven Gruppen der Komponente (c) oder den Aminogruppen der Komponente (b) oder durch Reaktion der gegenüber Isocyanat reaktiven Gruppen der Komponenten (c) mit den Isocyanatgruppen der Komponente (a). Durch die von den Komponenten (c) und (d) abgeleiteten Struktureinheiten werden also die Struktureinheiten der allgemeinen Formel I unterbrochen oder abgeschlossen oder sie befinden sich zwischen einer aus Komponente (a) und (b) gebildeten Struktureinheit. Die Komponenten (c) und (d) dienen somit hauptsächlich der Molekulargewichtsregelung, denn die Komponenten (c) und (d) wirken hauptsächlich als der Kettenverlängerer oder Kettenabbrecher.

Die Komponenten (c) tragen Gruppen, die mit Isocyanatgruppen in einer Additionsreaktion reagieren können. Beispielsweise können allgemein übliche Substanzen eingesetzt werden, die durch ihre Reaktion mit Isocyanaten Urethan- oder Harnstoffgruppen erzeugen. Beispielsweise können aromatische, aliphatische oder araliphatische Verbindungen mit 1 bis 20 Kohlenstoffatomen verwendet werden, die Hydroxyl- und/oder Aminogruppen als gegenüber Isocyanaten reaktive Gruppen enthalten. Bevorzugt werden als Verbindungen, die mindestens zwei gegenüber Isocyanaten reaktive Gruppen aufweisen, organische Verbindungen mit mindestens zwei Hydroxylgruppen, mit mindestens zwei Aminogruppen und/oder mindestens einer Hydroxylgruppe und mindestens einer Aminogruppe eingesetzt. Beispielsweise können verwendet werden: aromatische, araliphatische und/oder aliphatische Polyole mit 2 bis 20 Kohlenstoffatomen, bevorzugt solche mit primären Hydroxylgruppen. Z.B. können genannt werden: Ethandiol-1,2, Propandiol-1,3, Propandiol-1,2, Butandiol-1,4, -2,4, und/oder -2,3, Pentandiol-1,5, Hexandiol-1,6, Heptandiol-1,7, Octandiol-1,8, Decandiol-1,10, Neopentylglykol, 2-Methylpropandiol-1,3, 2- und 3-Methylpentandiol-1,5, Polyethylenglykole, Polypropylenglykole, bevorzugt mit 2 Hydroxylgruppen, die Isomeren des Bis(hydroxy-methyl- oder -ethyl)benzols, Hydroxyalkylether von Dihydroxybenzolen, Trimethylolpropan, Glycerin, Pentaerythrit oder Zucker mit beispielsweise 4, 5 oder 6 Hydroxylgruppen.

Falls gegenüber Isocyanaten reaktive Verbindungen verwendet werden, die Ethylenoxideinheiten aufweisen, so sollte der Anteil an Ethylenoxideinheiten in den erfindungsgemäßen Carbodiimiden bevorzugt 1 bis 15 Gew.-%, bezogen auf das Gewicht der Carbodiimide, betragen.
Bevorzugt werden keine solchen Verbindungen verwendet.

Als Amine sind Amine mit mindestens zwei primären und/oder sekundären Aminogruppen zu verwenden. Beispielhaft sind zu nennen: Amine des Molekulargewichtsbereiches von 32 bis 500 g/mol, vorzugsweise von 60 bis 300 g/mol, welche mindestens zwei primäre, mindestens zwei sekundäre oder mindestens je eine primäre und eine sekundäre Aminogruppe enthalten. Beispiele hierfür sind Diamine wie Diaminoethan, Diaminopropan, Diaminobutan, Diaminopertan, Diaminohexan, Piperazin, 2,5-Dimethylpiperazin, 1-Amino-3,3,5-trimethyl-5-aminomethyl-cyclohexan (Isophorondiamin, IPDA), 4,4'-Diaminodicyclohexylmethan, 1,4-Diaminocyclohexan, Aminoethylethanolamin, Hydrazin, Hydrazinhydrat oder Triamine wie Diethylentriamin, oder 1,8-Diamino-4-aminomethyloctan.

Weiterhin können Amine verwendet werden, die sich von den genannten Aminen dadurch ableiten, daß eine oder mehrere primäre Aminogruppen durch weitere Substituenten wie z.B. Alkylgruppen zu sekundären Aminogruppen substituiert werden. Des weiteren können auch Verbindungen, die sowohl mindestens eine Hydroxylgruppe als auch mindestens eine Aminogruppe aufweisen, eingesetzt werden, beispielsweise Ethanolamin, Propanolamin, Isopropanolamin, Aminoethylethanolamin oder davon abgeleitete N-Alkylamine.

Bevorzugt werden lineare Alkohole, Amine oder Aminoalkohole, besonders bevorzugt solche mit einer geraden Zahl von C-Atomen verwendet. Weiterhin bevorzugt sind Alkohole, Amine oder Aminoalkohole mit cyclischen Strukturelementen.

Gegebenenfalls kann es zweckmäßig sein, zusätzlich zu den beschriebenen gegenüber Isocyanaten reaktiven Verbindungen mit mindestens zwei funktionellen Gruppen auch monofunktionelle Verbindungen einzusetzen, um das Molekulargewicht der erfindungsgemäßen Carbodiimide zu regulieren, insbesondere wenn die Diisocyanate in einem ersten Schritt zu den Carbodiimiden umgesetzt werden und anschließend die Reaktion der Isocyanatgruppen-enthaltenden Carbodiimide mit den gegenüber Isocyanaten reaktiven Verbindungen erfolgt. Als monofunktionelle gegenüber Isocyanaten reaktive Verbindungen können beispielsweise Amine und bevorzugt Alkohole verwendet werden. Geeignete Amine, z.B. primäre oder vorzugsweise sekundäre Amine, besitzen vorteilhafterweise 1 bis 12 C-Atome, vorzugsweise 2 bis 8 C-Atome. Beispielhaft genannt seien Methyl-, Ethyl-, Propyl-, Butyl-, Pentyl-, Hexyl-, 2-Ethylhexyl-, Octyl-, Decyl-, Diethyl-, Dipropyl-, Dibutyl-, Methylbutyl-, Ethylbutyl- und Ethylhexylamin sowie Cyclohexyl- und Benzylamin. Zur Absättigung der Isocyanatgruppen finden jedoch vorzugsweise Alkohole, z.B. primäre oder sekundäre Alkohole mit 1 bis 18 C-Atomen, vorzugsweise 2 bis 8 C-Atomen Verwendung. Als primäre oder sekundäre Alkohole seien beispielhaft genannt: Methanol, Ethanol, n-Propanol, Isopropanol, n-Butanol, sekundär-Butanol, n-Pentanol, technische Pentanolmischungen, n-Hexanol, technische Hexanolgemische, 2-Ethylhexanol, Octanol, 2-Ethyloctanol, Dekanol und Dodekanol sowie Cyclohexanol und Benzylalkohol.

Bevorzugt wird die Komponente (b) mit monofunktionellen Verbindungen, besonders bevorzugt Monoaminen eingesetzt.

Im allgemeinen beträgt das Molekulargewicht der Komponenten (c) weniger als 400, insbesondere sind die erfindungsgemäßen Carbodiimide frei von Einheiten, die sich von Makropolyolen wie Polyetherpolyolen oder Polyesterpolyolen mit einem Molekulargewicht von mehr als 400 ableiten.

Als Komponente (d) kommen hauptsächlich aromatische Isocyanate in Betracht, z.B. 1,4-Diisocyanatobenzol, 2,4-Diisocyanatotoluol, 2,6-Diisocyanatotoluol, 4,4'-Diisocyanato-diphenylmethan oder 2,4'-Diisocyanato-diphenylmethan.

Im allgemeinen beträgt der Anteil der Komponenten (c) und (d), bezogen auf den Anteil aller Komponenten (a) bis (d), die zur Herstellung der Verbindungen V eingesetzt werden, nicht mehr als 0 bis 40, bevorzugt 0 bis 30 Gew.-%.

Die Herstellung der erfindungsgemäßen Carbodiimide erfolgt im wesentlichen durch zwei Umsetzungsschritte, indem man
I. Carbodiimide mit endständigen Isocyanatgruppen herstellt, indem man einen Teil der Isocyanatgruppen der Komponente (a) carbodimidisiert und
II. die gemäß Schritt I hergestellten Verbindungen mit endständigen Isocyanatgruppen mit der Komponente (b) und gegebenenfalls den Komponenten (c) und (d) umsetzt.

In Schritt I werden durch allgemein bekannte Umsetzung der Isocyanatgruppen miteinander unter Abspaltung von Kohlendioxid in Gegenwart von üblichen Katalysatoren, die für diese Umsetzung bekannt sind, Carbodiimidstrukturen erzeugt, In Schritt II werden Isocyanatgruppen mit gegenüber Isocyanaten reaktiven Verbindungen zur Herstellung von Urethan- und/oder Harnstoffstrukturen in bekannter Weise umgesetzt.

Das molare Verhältnis der NCO-Gruppen des Isocyanatgruppen-aufweisenden Carbodiimides zu der Summe aus den gegenüber Isocyanaten reaktiven Gruppen der Komponente (c) und den Aminogruppen der Komponente (a) beträgt üblicherweise 10:1 bis 0,2:1, bevorzugt 5:1 bis 0,5:1.

Alternativ können die erfindungsgemäßen Carbodiimide dadurch erhalten werden, daß man Komponente (a) zunächst mit den Komponenten (b) und gegebenfalls (c) umsetzt, wobei das Verhältnis von eingesetzten Isocyanatgruppen zu der Summe aus den gegenüber Isocyanaten reaktiven Gruppen der Komponente (c) und den Aminogruppen der Komponente (b) mindestens 2 : 1 beträgt, und anschließend das Isocyanatgruppen aufweisende Reaktionsprodukt in Gegenwart von Katalysatoren unter Kohlendioxidfreisetzung zu Carbodiimiden umsetzt. Nach dieser Verfahrensvariante werden zunächst bis zu 50 Gew.-%, vorzugsweise bis zu 23 Gew.-% der Isocyanatgruppen der Komponente (a) mit den gegenüber Isocyanaten reaktiven Verbindungen umgesetzt und danach die freien Isocyanatgruppen in Gegenwart von Katalysatoren unter Kohlendioxidfreisetzung ganz oder teilweise zu Carbodiimidgruppen umgesetzt.

Die Umsetzungen können bevorzugt in Gegenwart eines Lösungsmittels durchgeführt werden. Als Lösungsmittel sind vor allem solche Verbindungen geeignet, die das Reaktionsprodukt der Umsetzung gemäß Schritt I gut lösen und außerdem mit Wasser mischbar sind, beispielsweise Methanol, Ethanol, n- und/oder iso-Propanol, Propanon, Tetrahydrofuran, Dioxan, N-Methylpyrrolidon, Dimethylformamid, Dimethylacetamid und/oder Propylencarbonat. Bevorzugt werden Lösemittel mit einem Siedepunkt bei 1013 mbar von weniger als 100 °C verwendet.

Der Verfahrensschritt, in dem die Carbodiimidgruppen gebildet werden, kann bei erhöhten Temperaturen, z.B. bei Temperaturen von 50 bis 200°C, vorzugsweise von 150 bis 185°C, zweckmäßigerweise in Gegenwart von Katalysatoren durchgeführt werden. Hierfür geeignete Verfahren werden beispielsweise beschrieben in der GB-A-1 083 410, der DE-B 1 130 594 (GB-A-851 936) und der DE-A-11 56 401 (US-A-3 502 722). Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen besitzt, wird die Polycarbodiimidbildung üblicherweise beendet. Hierzu können die Katalysatoren unter vermindertem Druck abdestilliert oder durch Zusatz eines Desaktivatörs, wie z.B. Phosphortrichlorid, desaktiviert werden. Die Polycarbodiimidherstellung kann ferner in Abwesenheit oder Gegenwart von unter den Reaktionsbedingungen inerten Lösungsmitteln durchgeführt werden.

Die Temperatur bei dem Schritt, in dem vorwiegend Urethan- und Harnstoffgruppen gebildet werden, beträgt üblicherweise 10 bis 100 °C.

Wird Komponente (a) zuerst zu einem Isocyanatgruppen aufweisenden Carbodiimid (Schritt I) und anschließend zur Verbindung (V) umgesetzt, so weist das in Schritt I gebildete Zwischenprodukt bevorzugt einen NCO-Gehalt von 1 bis 18 Gew. % auf.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder CO₂-Entwicklung kann man für die Verfolgung des Ablaufs und die Steuerung der Reaktion heranziehen.

Die erfindungsgemäße Verbindung (V) eignet sich vor allem zur Molekulargewichtserhöhung von Polymeren (P), die in Form einer wässrigen Dispersion vorliegen.

Als Polymer (P), kommen praktisch alle filmbildenden Polymerisate in Betracht.

Bevorzugt tragen die Polymere (P) Carboxylgruppen, im allgemeinen in Mengen von 0,01 bis 2 mol/kg.

Mischungen aus Verbindungen (V) und wässrigen Dispersionen enthaltend Polymer (P) enthalten Verbindungen (V) und Polymer (P) bevorzugt im Gewichtsverhältnis 0,005 : 1 bis 1 : 1.

Die Vermischung ist unkritisch und kann beispielsweise so vorgenommen werden, daß man Verbindung (V) in die wässrige Dispersionen enthaltend Polymer (P) einrührt. Die Vermischung kann zu einem beliebigen Zeitpunkt vor deren Anwendung erfolgen.

Geeignete Polymere (P) sind beispielsweise wasserdispergierbare Polyurethane (Polymere PII). Derartige Polyurethane und die diese enthaltenen Dispersionen sind allgemein bekannt (vgl. Ullmann's Encyclopedia of Industrial Chemistry, 5^{th} Edition, Volume A 21, Seiten 677f).

Bevorzugt sind derartige Dispersionen aufgebaut aus
IIa) Diisocyanaten mit 4 bis 30 C-Atomen,
IIb) Diolen, von denen
   IIb1) 10 bis 100 mol-%, bezogen auf die Gesamtmenge der Diole (IIb), ein Molekulargewicht von 500 bis 5000 aufweisen, und
   IIb2) 0 bis 90 mol-%, bezogen auf die Gesamtmenge der Diole, ein Molekulargewicht von 60 bis 500 g/mol aufweisen,
IIc) von den Monomeren (IIa) und (IIb) verschiedene Monomere mit wenigstens einer Isocyanatgruppe oder wenigstens einer gegenüber Isocyanatgruppen reaktiven Gruppe, die darüberhinaus wenigstens eine hydrophile Gruppe oder eine potentiell hydrophile Gruppe tragen, wodurch die Wasserdispergierbarkeit der Polyurethane bewirkt wird,
IId) gegebenenfalls weiteren von den Monomeren (IIa) bis (IIc) verschiedenen mehrwertigen Verbindungen mit reaktiven Gruppen, bei denen es sich um alkoholische Hydroxylgruppen, primäre oder sekundäre Aminogruppen oder Isocyanatgruppen handelt und
IIe) gegebenenfalls von den Monomeren (IIa) bis (IId) verschiedenen einwertigen Verbindungen mit einer reaktiven Gruppe, bei der es sich um eine alkoholische Hydroxylgruppe, eine primäre oder sekundäre Aminogruppe oder eine Isocyanatgruppe handelt.

Als Monomere (IIa) kommen die üblicherweise in der Polyurethanchemie eingesetzten aliphatischen oder aromatischen Diisocyanate in Betracht. Bevorzugt sind die Monomere (IIa) oder deren Mischungen, die auch als Monomere (IIa) in der DE-A-19521500 erwähnt sind.

Als Monomere (IIb) und (IId) kommen bevorzugt die in der DE-A-19521500 als Monomere (IIb) und (IId) genannten in Betracht.

Monomere IIb1 sind beispielsweise Polyester- oder Polyetherdiole.

Bei den Monomeren IIb2 handelt es sich beispielsweise um aliphatische Diole mit 2 bis 12 C-Atomen, z.B. 1,4-Butandiol oder 1,6-Hexandiol.

Als Monomere (IId) sind beispielsweise aliphatische Amine mit 2 bis 12 C-Atomen und 2 bis 4 Gruppen, ausgewählt aus der Gruppe der primären oder sekundären Aminogruppen, geeignet. Beispiele sind Ethylendiamin, Isophorondiamin oder Diethylentriamin.

Um die Wasserdispergierbarkeit der Polyurethane zu erreichen, sind die Polyurethane neben den Komponenten (IIa), (IIb) und (IId) aus von den Komponenten (IIa), (IIb) und (IId) verschiedenen Monomere (IIc), die wenigstens eine Isocyanatgruppe oder wenigstens eine gegenüber Isocyanatgruppen reaktive Gruppe und darüberhinaus wenigstens eine hydrophile Gruppe oder eine Gruppe, die sich in eine hydrophile Gruppe überführen läßt, tragen, aufgebaut. Im folgenden Text wird der Begriff "hydrophile Gruppen oder potentiell hydrophile Gruppen" mit "(potentiell) hydrophile Gruppen" abgekürzt. Die (potentiell) hydrophilen Gruppen reagieren mit Isocyanaten wesentlich langsamer als die funktionellen Gruppen der Monomere, die zum Aufbau der Polymerhauptkette dienen.

Bevorzugte Monomere (IIc) sind ebenfalls die in der DE-A-19521500 als Monomere (IIc) bezeichneten.

Der Anteil der Komponenten mit (potentiell) hydrophilen Gruppen an der Gesamtmenge der Komponenten (IIa), (IIb), (IIc), (IId) und (IIe) wird im allgemeinen so bemessen, daß die Molmenge der (potentiell) hydrophilen Gruppen, bezogen auf die Gewichtsmenge aller Monomere (a) bis (e), 80 bis 1200, bevorzugt 100 bis 1000 und besonders bevorzugt 150 bis 800 mmol/kg beträgt.

Bei den (potentiell) hydrophilen Gruppen kann es sich um nichtionische, z.B. Polyethylenoxidgruppen oder bevorzugt um (potentiell) ionische hydrophile Gruppen, z.B. Carboxylat- oder Sulfonat-Gruppen handeln. Bevorzugt wird ohne wirksame Mengen an nichtionischen Gruppen gearbeitet

Der Gehalt an nichtionischen hydrophilen Gruppen, falls solche eingebaut werden, beträgt im allgemeinen bis 5, bevorzugt bis 3, besonders bevorzugt bis 1 Gew.-%, bezogen auf die Gewichtsmenge aller Monomere (IIa) bis (IIe).

Monomere (IIe), die gegebenenfalls mitverwendet werden, sind Monoisocyanate, Monoalkohole und mono-primäre und -sekundäre Amine. Im allgemeinen beträgt ihr Anteil maximal 10 mol-%, bezogen auf die gesamte Molmenge der Monomere. Diese monofunktionellen Verbindungen tragen üblicherweise weitere funktionelle Gruppen wie Carbonylgruppen und dienen zur Einführung von funktionellen Gruppen in das Polyurethan, die die Dispergierung bzw. die Vernetzung oder weitere polymeranaloge Umsetzung des Polyurethans ermöglichen.

Auf dem Gebiet der Polyurethanchemie ist allgemein bekannt, wie das Molekulargewicht der Polyurethane durch Wahl der Anteile der miteinander reaktiven Monomere sowie dem arithmetischen Mittel der Zahl der reaktiven funktionellen Gruppen pro Molekül eingestellt werden kann.

Normalerweise werden die Komponenten (IIa) bis (IIe) sowie ihre jeweiligen Molmengen so gewählt, daß das Verhältnis A : B mit
A) der Molmenge an Isocyanatgruppen und
B) der Summe aus der Molmenge der Hydroxylgruppen und der Molmenge der funktionellen Gruppen, die mit Isocyanaten in einer Additionsreaktion reagieren können
0,5 : 1 bis 2 : 1, bevorzugt 0,8 : 1 bis 1,5, besonders bevorzugt
0,9 : 1 bis 1,2 : 1 beträgt. Ganz besonders bevorzugt liegt das Verhältnis A : B möglichst nahe an 1 : 1.

Weiterhin wird der Anteil der Monomere (a) bevorzugt so gewählt, daß der Anteil der Monomere (IIa) an den Monomeren (IIa) bis (IIe) 20 bis 70 Gew.-% beträgt.

Die eingesetzten Monomere (IIa) bis (IIe) tragen im Mittel üblicherweise 1,5 bis 2,5, bevorzugt 1,9 bis 2,1, besonders bevorzugt 2,0 Isocyanatgruppen bzw. funktionelle Gruppen, die mit Isocyanaten in einer Additionsreaktion reagieren können.

Die verschiedenen Herstellmethoden der Polymere PII sind allgemein bekannt und beispielsweise in der DE-A-19807754 näher beschrieben.

Weiterhin kann es sich bei den Polymeren (P) um übliche Emulsonspolymerisate (Polymere PIII) handeln.

Diese sind im allgemeinen aufgebaut aus
IIIa) 30 bis 99,9 Gew.-% Hauptmonomere ausgewählt aus C₁- bis C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden und aliphatischen Kohlenwasserstoffen mit 2 bis 8 C-Atomen und 1 oder 2 Doppelbindungen,
IIIb) 0 bis 20, bevorzugt 0,01 bis 20 Gew.-% einer Carbonsäure mit einer olefinischen Doppelbindung und
IIIc) 0 bis 20 Gew.-% von (IIIa) und (IIIb) verschiedenen radikalisch polymerisierbaren Monomeren.

Als Monomere (IIIa) zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinylpropionat und Vinylacetat.

Als vinylaromatische Verbindungen kommen Vinyltoluol, alpha- und p-Methylstyrol, alpha-Butylstyrol, 4-n-Butylstyrol, 4-n-Decylstyrol und vorzugsweise Styrol in Betracht.

Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und ein oder zwei olefinischen Doppelbindungen seien Butadien, Isopren, und Chloropren, sowie Ethylen, Propylen und Isobutylen genannt.

Die Hauptmonomeren (IIIa) werden auch vorzugsweise im Gemisch eingesetzt.

Vinylaromatische Verbindungen wie Styrol werden z.B. häufig im Gemisch mit C₁-C₂₀-Alkyl(meth)acrylaten, insbesondere mit C₁-C₈-Alkyl(meth)acrylaten, oder nicht aromatischen Kohlenwasserstoffen wie Isopren oder vorzugsweise Butadien eingesetzt.

Als Monomere (IIIb) kommen vorzugsweise (Meth)acrylsäure oder Maleinsäure in Betracht.

Als Monomere (IIIc) kommen z.B. in Betracht: Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, wie 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder Cycloalkylester, wie Cyclohexyl(meth)acrylat, Phenylethyl-(meth)acrylat, Phenylpropyl-(meth)acrylat oder Acrylsäureester von heterocyclischen Alkoholen wie Furfuryl(meth)acrylat genannt.

Darüber hinaus kommen als Monomer (IIIc) noch Monomere mit Amino- oder Amidgruppen wie (Meth)acrylamid, sowie deren am Stickstoff mit C₁-C₄-Alkyl substituierten Derivate, in Betracht.

Von Bedeutung als Monomere (IIIc) sind insbesondere hydroxyfunktionelle Monomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₂-C₈- hydroxyalkylester, wie n-Hydroxyethyl-, n-Hydroxypropyl- oder n-Hydroxybutyl(meth)acrylat.

Die Herstellung des Polymers (PIII) erfolgt durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid mit Fettalkoholen, -säuren oder Phenolen, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak, zu Carbonsäuregruppen enthaltenden Copolymerisaten in Wasser ohne Verwendung eines Emulgators oder Dispergierhilfsmittels dispergiert. Das organische Lösungsmittel kann abdestilliert werden. Die Herstellung von wäßrigen Sekundärdispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan, sie können z.B. in Mengen von 0 bis 0,5 Gew.-%, bezogen auf das Copolymerisat, zusätzlich eingesetzt werden.

Die Art und Menge der Comonomeren wird vorzugsweise so gewählt, daß das erhaltene Copolymerisat eine Glasübergangstemperatur von -60 bis +140°C, vorzugsweise -60 bis +100°C aufweist. Die Glasübergangstemperatur des Copolymerisats wird durch Differentialthermoanalyse oder Differential Scanning Calorimetrie nach ASTM 3418/82, bestimmt.

Das zahlenmittlere Molekulargewicht Mₙ beträgt vorzugsweise 10³ bis 5·10⁶, besonders bevorzugt 10⁵ bis 2·10⁶ g/mol (bestimmt durch Gelpermeationschromatographie mit Polystyrol als Standard).

Weiterhin kann es sich bei den Polymeren (P) um (Polymere PIV) um einen wasserdispergierbaren Polyester, der Carboxylgruppen trägt, handeln.

Die wasserdispergierbaren Polyester, die Carboxylgruppen tragen, (Polymer IV), sind beispielsweise aus Encyclopedia of polymer science and engineering, John Wiley & Sons, second edition, volume 12, Seiten 300 bis 313 bekannt.

Die wässrigen Dispersionen, die das Polymer (P) enthalten, weisen üblicherweise einen Feststoffgehalt von 10 bis 70 Gew.-% auf.

Die erfindungsgemäßen Mischungen enthaltend Verbindung (V) und Polymer (P) können handelsübliche Hilfs- und Zusatzstoffe wie Netzmittel, Entschäumer, Mattierungsmittel, Emulgatoren, Verdikkungsmittel und Thixotropiermittel, Farbmittel wie Farbstoffe und Pigmente enthalten.

Sie eignen sich beispielsweise zum Verkleben oder Beschichten unterschiedlicher Substrate wie Holz, Metall, Kunststoff, Papier, Leder oder Textil, für die Imprägnierung von Textilien sowie für die Herstellung von Formkörpern und Druckfarben.

Die Verarbeitung der erfindungsgemäßen Dispersionen kann dabei nach den in der Klebstoff, Leder- oder Lackindustrie allgemein üblichen Verfahren erfolgen, also indem man die Dispersionen auf das Substrat sprüht, walzt oder rakelt und anschließend trocknet.

Für den Fall der Verarbeitung als Klebstoff werden die beschichteten Werkstücke entweder vor dem Trocknen des Dispersionsfilms oder nach dem Trocknen mit einem anderen Werkstück bevorzugt unter Anwendung von Druck zusammengefügt.

Besonders feste Klebstoffverbunde erhält man, wenn man Werkstücke, die mit einem getrockneten Klebstofffilm versehen sind, unmittelbar vor, während oder nach dem Zusammenfügen auf eine Temperatur von ca. 50 bis 100°C erwärmt.

Die nach diesen Methoden hergestellten Klebstoffverbunde zeichnen sich insbesondere dadurch aus, daß sie lagerbeständig sind und sich mit ihnen Verklebungen mit einer hohen Wärmestandfestigkeit herstellen lassen.

Weiterhin lassen sich die Verbindungen V zur Herstellung von Klebstofffolien einsetzen. Hierzu werden wässrige Dispersionen enthaltend ein Polymer (PII) oder (PIII) mit der Verbindung (V) abgemischt. Diese Mischung wird nach den üblichen vorgenannten Methoden auf Kunststoffolien aufgetragen, wobei coronabehandelte Polyethylenfolie bevorzugt ist. Die Auftragsmengen betragen üblicherweise 5 bis 6 g/m².

Beschichtete Klebstofffolie aus coronabehandelter Polyethylenfolie eignet sich zum Bekleben von Gegenständen aller Art. Wird eine solche Folie mit einer Mischung aus einer Verbindung (V) und einem Polymer (PII) oder (PIII) eingesetzt, das als Haftklebstoff einsetzbar ist, so zeichnet sich die beschichtete Folie insbesondere dadurch aus, daß sie sich samt Polymer (PII) oder (III) rückstandsfrei vom Substrat lösen läßt. Die Tatsache, daß die Klebstoffschicht, gebildet aus Polymer (PII) oder (III), besser auf der Polyethylenfolie als auf dem Substrat haftet und eine hohe Kohäsion aufweist, führen wir darauf zurück, daß die Verbindung (V) das Molekulargewicht des Polymeren (PII) oder (III) erhöht und es gleichzeitig auf der Polyethylenfolie unter Ausbildung von kovalenten Bindungen verankert, indem die Carbodiimidgruppen der Verbindung (V) vermutlich mit den Carboxylgruppen auf der Oberfläche der Polyethylenfolie, die bei der Coronabehandlung entstehen, reagieren.

Derartige Klebstofffolien eignen sich deshalb insbesondere zur Herstellung von Etiketten oder für die Verwendung als Schutzfolien, um Gegenstände, insbesondere solche mit empfindlichen Oberflächen wie lackierte Oberflächen oder solche aus Plexiglas, Polycarbonat oder Glas, z.B. Bildschirme oder Scheiben, bei der Lagerung und Transport vor mechanischen Beschädigungen, z.B. Verkratzen, oder sonstigen Umwelteinflüssen zu schützen. Sie besitzen zusätzlich den Vorteil, daß sie einen guten "Tack" aufweisen, d.h. daß die Folie bei bloßer Berührung ohne Anwendung von hohem Druck, z.B. durch Aufstreichen mit der Hand oder durch Auflegen der Folie, bereits auf dem Substrat haftet und sich mit mäßiger Kraft (z.B. unter Anwendung von 1,25 bis 2,5 N bei einem Klebstoffstreifen mit einer Breite von 25 mm) wieder vom Substrat abschälen lassen.

### Experimenteller Teil

### 1. Herstellung der Carboxyl-Carbodiimide

### 1.1. mit Dimethylolpropionsäure

In eine Lösung aus 67 g (0,5 mol) DMPA und 60,0 g (0,593 mol) Triethylamin (TEA) in 100 g Aceton wurden unter Rühren eine Lösung aus 500 g eines NCO-terminierten Carbodiimids aus TMXDI mit einem NCO-Gehalt von 7,8 Gew.% in 100 g Aceton gegeben. Nach 240 min Rühren bei 60°C wurde mit 2000 g Wasser verdünnt und das Aceton im Vakuum abgezogen.

Man erhält eine kolloidale, wässrige Lösung eines Carbodiimids mit 22% Festgehalt und einem LD-Wert von 100.

### 1.2. mit Hydroxypivalinsäure

In eine Lösung aus 59 g (0,5 mol) Hydroxypivalinsäure und 60,0 g (0,593 mol) Triethylamin (TEA) in 100 g Aceton wurden unter Rühren eine Lösung aus 250 g eines NCO-terminierten Carbodiimids aus TMXDI mit einem NCO-Gehalt von 7,8 Gew.% in 50 g Aceton gegeben. Nach 240 min Rühren bei 60°C wurde mit 1200 g Wasser verdünnt und das Aceton im Vakuum abgezogen. Man erhält eine kolloidale, wässrige Lösung eines Carbodiimids mit 23% Festgehalt und einem LD-Wert von 100.

### 2. Anwendungsbeispiele

### Anwendungsbeispiel 2.1: Verwendung bei der Lederzurichtung

Zur Prüfung als Lederzurichtung wurde eine Spritzflotte folgender Zusammensetzung hergestellt:
45,7 T. Astacin® Top GA
45,5 T. vollentsalztes Wasser
0,5 T. Lepton® Paste VL
1,0 T. Lepton® Wachs LD6609.

Astacin Top GA ist eine Polyester-Polyurethandispersion mit einem Feststoffgehalt von 30 Gew.%.

Lepton Paste VL ist ein Assoziativverdicker auf Urethan-Basis.

Lepton Wachs LD 6609 ist ein Griffmittel auf Silikonbasis.

Dieser Flotte wurden in Anwendungsbeispiel A1.1. 7,8 T., in Anwendungsbeispiel A1.2. 15,5 T. der Lösung aus Beispiel 1.1. zugesetzt.

Im Vergleichsbeispiel V1 wurde keine Lösung zugesetzt.

Die Flotten wurden mit einer Auftragsmenge von 10 g/DIN A4 auf grundiertes Rindboxleder gespritzt und bei A) 10 min/80°C und B) 12h/80°C getrocknet.

Die Leder wurden bei 23°C und 50% relativer Luftfeuchte 2 Tage lang konditioniert und dann einer physikalischen Lederprüfung mit folgenden Ergebnissen unterzogen:

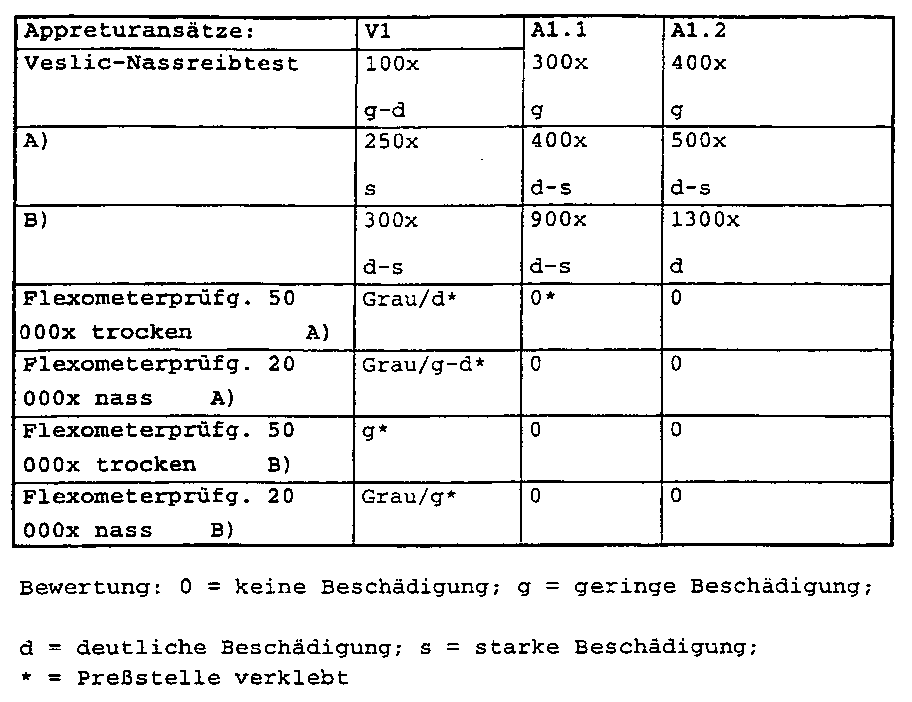

### Anwendungsbeispiel 2.2: Verwendung zur Herstellung von Schutzfolien

Herstellung einer Polymerdispersion:

| | Vorlage (g) | Monomer-Emulsion (g) | Initiator (g) | Nachkatalyse |
|---|---|---|---|---|
| Wasser | 260 | 200 | | 35,10 |
| Emulgatorlösung 1 | | 24 | | |
| Emulgatorlösung 2 | | 8 | | |
| 2-EHA | | 214,5 | | |
| nBA | | 450,45 | | |
| MMA | | 21,45 | | |
| M-Amol | | 95,33 | | |
| AA | | 14,3 | | |
| NaPS 5% ige Lösung in Wasser | | | 71,5 | |
| NH₃ 25%ige Lösung in Wasser | | | | 13,5 |
| t-BHP 10%ige Lösung in Wasser | | | | 21,45 |
| Aceton | | | | 1,36 |
| Na-Disulfit | | | | 2,15 |

Emulgatorlösung 1: 30 gew-%ige Lösung des Natriumsalzes eines Schwefelsäurehalbestergemisches von C10-C16 Alkyl-Ethoxylaten (mittlerer EO-Grad 30) in Wasser (Disponil® FES 77 der Henkel KgaG)

Emulgatorlösung 2: 45 gew-%ige Lösung von (Dodecyl-sulfonyl-phenoxy)benzolsulfonsäurenatriumsalz (Dowfax® 2A1 der Dow Chemicals)

### Fahrweise:

Die Vorlage wurde in einen 2-Liter-Kolben mit Rückflußkühler, Stickstoffeinleitung und Metallrührer gefüllt. Unter Stickstoffspülung wurde auf 90°C gebracht. 20% der Initiatorlösung wurden zugegeben. Nach 5 Minuten wurde die Monomer-Emulsion innerhalb von 3 Stunden zugegeben. Gleichzeitig wurde die restliche Initiator-Lösung innerhalb von 3,5 Stunden zugegeben. Nach dem Ende des Initiator-Zulaufes wurde für 30 Minuten nachpolymerisiert. Dann wurde auf 80°C gekühlt und die Nachkatalysator-Lösung und der Ammoniak zugegeben.

Die beiden Zuläufe t-BHP und Aceton/Na-Disulfit wurden parallel innerhalb einer Stunde zugegeben. Danach wurde die Dispersion auf Raumtemperatur abgekühlt.

Der Festgehalt der Dispersion beträgt 51%; der pH-Wert liegt bei 7. Die Teilchengröße liegt bei 280 nm (bestimmt mittels Malvern Autosizer).

### Anwendung: Schutzfolie

### Herstellung der Schutzfolien

Die Dispersion wurde mit 1,5% (fest/fest) (A2.1.) bzw. mit 3% (fest/fest) (A2.2.) der Lösung aus Beispiel 1.1. gemischt. Zum Vergleich wurde eine Mischung aus der Dispersion mit 1,5% Basonat® FDS 3425 hergestellt (V2).

Die Mischungen wurden auf eine corona-behandelte Polyethylenfolie mit 5 bis 6 g/m2 aufgerakelt und bei 90°C drei Minuten lang getrocknet. Die Folien wurden mit Silikonpapier abgedeckt und drei Tage bei Raumtemperatur gelagert.

### Prüfung auf rückstandfreies Abziehen:

Dieser Test besteht in der Bewertung des Aussehens der zu schützenden Oberfläche nach dem Abziehen der Schutzfolie. Die mit der Folie beklebte Oberfläche wird eine und vier Wochen bei 50°C und 80% relativer Luftfeuchte gelagert. Dann werden die Folien von Hand einmal langsam und einmal rasch abgezogen und der Rückstand auf der Oberfläche per Augenschein beurteilt. Im optimalen Falle ist die Oberfläche frei von Rückständen des Klebstoffs.

Bewertet wird nach folgender Skala:
1 Kein Rückstand
1* Negativ-Abdruck der Schutzfolie (Schatten am Rand, sonst keine Schatten)
2 Schatten der Schutzfolie
3 Rückstand erkennbar
4 Partieller Transfer des Klebstoffes
5 Vollständiger Transfer des Klebstoffes
6 Kohäsionsbruch

Das Abziehverhalten wird nach folgender Skala beurteilt:
A leicht klebrig
B leicht abziehbar
C schwer abziehbar

Geprüft wurde auf Stahl, Polycarbonat und Plexiglas.

Optimal ist die Note B1.

**Tabelle:**

| Ergebnisse der Abzugprüfung. | | | | | | | |
|---|---|---|---|---|---|---|---|
| Dispersion | Abzug | Plexiglas | | Polycarbonat | | Stahl | |
| | | 1 Woche | 4 Wochen | 1 Woche | 4 Wochen | 1 Woche | 4 Wochen |
| Dispersion ohne Vernetzer | langsam | B1 | B1 | B1 | B1 | B1* | B2 |
| | rasch | B1 | B1 | B1 | B1 | B3 | B3 |
| Dispersion | langsam | B1 | B1 | B1 | B1 | B2 | B2 |
| +1,5% Basonat FDS3425 | rasch | B1 | B1 | B1 | B1 | B2 | B3 |
| Dispersion Dispersion | langsam | B1 | B1 | B1 | B1 | B2 | B1* |
| +1,5% Lösung 1.1 | rasch | B1 | B1 | B1 | B1 | B2 | B2 |
| | langsam | B1 | B1 | B1 | B1 | B1* | B1* |
| + 3% Lösung 1.1 | rasch | B1 | B1 | B1 | B1 | B1* | B2 |

### Quickstick, Schälfestigkeit und Kratztest auf PE-Folie:

Die Dispersionen wurde auf 25 mm breite Stücke PE-Folie mit 20g/m2 aufgerakelt und drei Minuten bei 90°C getrocknet.

Die so erhaltenen Schutzfolien wurden auf eine Stahlplatte geklebt und "Quickstick" und Schälfestigkeit bei 23°C und 50% relativer Luftfeuchte geprüft.

Die Quickstick-Prüfung ist eine der bekanntesten Verfahren zur Tackmessung (Tack ist die Fähigkeit eines Haftklebstoffes, auf einer Oberfläche sofort zu haften). Bei der Quickstick Methode (FINAT Methode) wird eine Prüfstreifen zur Schlaufe gelegt, mit einer Glasplatte in Kontakt gebracht und unmittelbar danach wieder abgezogen.

Die Haftung der Dispersion auf der Folie wird mittels des Kratztestes geprüft: Je schwieriger der Film mit dem Finger abzukratzen ist, desto besser ist die Haftung der Dispersion.

| Schälrate: 300 mm/min | | | | | | | |
|---|---|---|---|---|---|---|---|
| | Quickstick N/25mm Stahl | | Schälfestigkeit in N/25mm Stahl | | | | Kratztest |
| | | | sofort | | 24 Stunden | | |
| Dispersion ohne Vernetzer | 3,5 | A | 1,8 | A | 6,2 | A | 3 |
| Dispersion + 1,5% Bas. FDS 3425 | 2,4 | A | 1,1 | A | 4,6 | A | 1 |
| Dispersion + 1,5% Carbodiimid 1.1 | 3,1 | A | 1,5 | A | 6,5 | A | 1 - 2 |
| Dispersion + 3,0% Carbodiimid 1.1 | 2,6 | A | 1,2 | A | 6,3 | A | 1 |
| Kratztest 1 = kein abkratzen 2 = schwierig abzukratzen 3 = leicht abzukratzen | | | | | | | |

### 3. Bestimmung der Vernetzungsdichte durch Messung des Dynamischen Schubmoduls von Filmen:

An Filmen mit und ohne Carbodiimid bzw. Basonat wurden der Speichermodul G' und der Verlustmodul G'' in Abhängigkeit von der Temperatur gemessen. Die Filme wurden 10 min bei 90°C getempert.

**Tabelle:**

| Speichermoduln bei 100°C | |
|---|---|
| Probe | G'bei 100°C (x 10⁴ Pa) |
| Dispersion ohne Vernetzer | 4,98 |
| Dispersion + 1,5% Bas. FDS 3425 | 6,46 |
| Dispersion + 1,5% Carbodiimid 1.1 | 5,81 |
| Dispersion + 3,0% Carbodiimid 1.1 | 7,17 |

Die Zusätze erhöhen den Speichermodul bei hohen Temperaturen, der ein Maß für die Vernetzungdichte ist.

### Abkürzungen

- nBA:: n-Butylacrylat
- MMA:: Methylmethacrylat
- M-Amol:: Methylolmethacrylamid
- AA:: Acrylsäure
- NaPS:: Natriumperoxodisulfat
- t-BHP:: tert.-Butylhydroperoxid
- NH₃:: Ammoniak
- 2-EHA:: 2-Ethylhexylacrylat
- T.:: Teile

## Patentansprüche

1. Verbindungen mit Carbodiimideinheiten und Carboxyl- oder Carboxylatgruppen (Verbindungen V), abgeleitet von
a) aliphatischen oder araliphatischen C₄- bis C₂₀-Polyisocyanaten (Komponente a)
b) Hydroxycarbonsäuren oder Hydroxycarbonsäuresalzen (Komponente b) und
c) gegebenenfalls weiteren Verbindungen, die Gruppen tragen, die mit Isocyanatgruppen in einer Additionsreaktion reagieren können (Komponente c)
d) gegebenenfalls sonstigen Isocyanaten (Komponente d)
wobei sich die Carbodiimideinheiten im wesentlichen ausschließlich von den Isocyanatgruppen der Komponente (a) ableiten.

2. Verbindungen (V) nach Anspruch 1, enthaltend 200 bis 2000 mmol/kg Carboxyl- oder Carboxylatgruppen, bezogen auf das Gewicht der Verbindungen.

3. Verbindungen (V) nach Anspruch 1 oder 2, wobei es sich bei der Komponente (a) um Hexamethylendiisocyanat oder 1,3-Bis-(1-methyl-1-isocyanato-ethyl)benzol handelt.

4. Verbindungen (V) nach den Ansprüchen 1 bis 3, wobei es sich bei den Hydroxycarbonsäuren bzw. Hydroxycarbonsäuresalzen um Carbonsäuren bzw. Salze mit einer oder zwei Hydroxylgruppen handelt.

5. Verbindungen (V) nach den Ansprüchen 1 bis 4, wobei es sich bei der Komponente (c) um aromatische, aliphatische oder gegebenenfalls Polyalkylenoxidgruppen tragende araliphatische Verbindungen mit 1 bis 20 Kohlenstoffatomen (wobei bei der genannten Zahl der Kohlenstoffatome die der Polyalkylenoxidgruppen nicht mitgezählt werden) und mindestens einer funktionellen Gruppe, ausgewählt aus der Gruppe der sekundären Aminogruppe, der primäre Aminogruppe oder der alkoholischen Hydroxylgruppe, handelt.

6. Verfahren zur Herstellung von Verbindungen (V) nach den Ansprüchen 1 bis 5, wobei man
I. Carbodiimide mit endständigen Isocyanatgruppen herstellt, indem man einen Teil der Isocyanatgruppen der Komponente (a) carbodimidisiert und
II. die gemäß Schritt I hergestellten Verbindungen mit endständigen Isocyanatgruppen mit der Komponente (b) und gegebenfalls den Komponenten (c) und (d) umsetzt.

7. Mischungen aus einer Verbindung (V) gemäß Anspruch 1 bis 5 und einer wässrigen Dispersion enthaltend ein Polymer (P).

8. Mischungen gemäß Anspruch 7, wobei das Polymer (P) Carboxylgruppen trägt.

9. Mischungen nach Anspruch 7, wobei es sich bei Polymer (P) um ein Polyurethan (PII), aufgebaut aus
IIa) Diisocyanaten mit 4 bis 30 C-Atomen,
IIb) Diolen, von denen
IIb1) 10 bis 100 mol-%, bezogen auf die Gesamtmenge der Diole (IIb), ein Molekulargewicht von 500 bis 5000 aufweisen, und
IIb2) 0 bis 90 mol-%, bezogen auf die Gesamtmenge der Diole (IIb), ein Molekulargewicht von 60 bis 500 g/mol aufweisen,
IIc) von den Monomeren (IIa) und (IIb) verschiedene Monomere mit wenigstens einer Isocyanatgruppe oder wenigstens einer gegenüber Isocyanatgruppen reaktiven Gruppe, die darüber hinaus wenigstens eine hydrophile Gruppe oder eine potentiell hydrophile Gruppe tragen, wodurch die Wasserdispergierbarkeit der Polyurethane bewirkt wird,
IId) gegebenenfalls weiteren von den Monomeren (IIa) bis (IIc) verschiedenen mehrwertigen Verbindungen mit reaktiven Gruppen, bei denen es sich um alkoholische Hydroxylgruppen, primäre oder sekundäre Aminogruppen oder Isocyanatgruppen handelt, und
IIe) gegebenenfalls von den Monomeren (IIa) bis (IId) verschiedenen einwertigen Verbindungen mit einer reaktiven Gruppe, bei der es sich um eine alkoholische Hydroxylgruppe, eine primäre oder sekundäre Aminogruppe oder eine Isocyanatgruppe handelt.

10. Mischungen nach Anspruch 7, wobei es sich bei Polymer (P) um ein Polymer (PIII), aufgebaut aus
IIIa) 30 bis 99,9 Gew.-% Hauptmonomere ausgewählt aus C₁- bis C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden und aliphatischen Kohlenwasserstoffen mit 2 bis 8 C-Atomen und 1 oder 2 Doppelbindungen,
IIIb) 0 bis 20 Gew.-% einer Carbonsäure mit einer olefinischen Doppelbindung und
IIIc) 0 bis 20 Gew.-% von (IIIa) und (IIIb) verschiedenen radikalisch polymerisierbaren Monomeren.

11. Gegenstände, die mit einer Mischung gemäß den Ansprüchen 7 bis 9 verklebt oder beschichtet sind, sowie Textilien, die mit dieser Mischung imprägniert sind.

## Claims

1. A compound with carbodiimide units and carboxyl or carboxylate groups (compound V), derived from
a) aliphatic or araliphatic C₄ to C₂₀ polyisocyanates (component a)
b) hydroxy carboxylic acids or hydroxy carboxylic salts (component b) and
c) if desired, further compounds, carrying groups able to react with isocyanate groups in an addition reaction (component c)
d) if desired, other isocyanates (component d),
the carbodiimide units being derived essentially exclusively from the isocyanate groups of component a).

2. A compound (V) as claimed in claim 1, comprising from 200 to 2000 mmol/kg of carboxyl or carboxylate groups, based on the weight of the compound.

3. A compound (V) as claimed in claim 1 or 2, wherein component (a) comprises hexamethylene diisocyanate or 1,3-bis(1-methyl-1-isocyanatoethyl)benzene.

4. A compound (V) as claimed in any of claims 1 to 3, wherein the hydroxy carboxylic acids or hydroxy carboxylic salts comprise carboxylic acids or salts, respectively, having one or two hydroxyl groups.

5. A compound (V) as claimed in any of claims 1 to 4, wherein component (c) comprises aromatic, aliphatic or, if desired, araliphatic compounds carrying polyalkylene oxide groups, having 1 to 20 carbon atoms (not including those of the polyalkylene oxide groups) and at least one functional group selected from the group consisting of secondary amino groups, primary amino groups, and alcoholic hydroxyl groups.

6. A process for preparing a compound (V) as claimed in any of claims 1 to 5, which comprises
I. preparing carbodiimides having terminal isocyanate groups, by carbodiimidizing some of the isocyanate groups of component (a), and
II. reacting the compounds prepared in step I, having terminal isocyanate groups, with component (b) and, if desired, components (c) and (d).

7. A mixture of a compound (V) as claimed in any of claims 1 to 5 and an aqueous dispersion comprising a polymer (P).

8. A mixture as claimed in claim 7, wherein the polymer (P) carries carboxyl groups.

9. A mixture as claimed in claim 7, wherein polymer (P) comprises a polyurethane (PII) composed of
IIa) Diisocyanates having 4 to 30 carbon atoms,
IIb) Diols of which
IIb1) from 10 to 100 mol%, based on the overall amount of the diols (IIb), have a molecular weight of from 500 to 5000, and
IIb2) from 0 to 90 mol%, based on the overall amount of the diols (IIb), have a molecular weight of from 60 to 500 g/mol,
IIc) monomers other than the monomers (IIa) and (IIb), which have at least one isocyanate group or at least one isocyanate-reactive group and also carry at least one hydrophilic group or one potentially hydrophilic group, which makes the polyurethanes dispersible in water,
IId) if desired, further, polyfunctional compounds which are different from the monomers (IIa) to (IIc) and have reactive groups which are alcoholic hydroxyl groups, primary or secondary amino groups, or isocyanate groups, and
IIe) if desired, monofunctional compounds which are different from the monomers (IIa) to (IId) and have a reactive group which is an alcoholic hydroxyl group, a primary or secondary amino group, or an isocyanate group.

10. A mixture as claimed in claim 7, wherein polymer (P) comprises a polymer (PIII) composed of
IIIa) from 30 to 99.9% by weight of principal monomers selected from C₁ to C₂₀ alkyl (meth)acrylates, vinyl esters of carboxylic acids comprising up to 20 carbon atoms, vinylaromatic compounds having up to 20 carbon atoms, ethylenically unsaturated nitriles, vinyl halides, and aliphatic hydrocarbons having 2 to 8 carbon atoms and 1 or 2 double bonds,
IIIb) from 0 to 20% by weight of a carboxylic acid having an olefinic double bond, and
IIIc) from 0 to 20% by weight of free-radically polymerizable monomers other than (IIIa) and (IIIb).

11. An article bonded or coated with the mixture as claimed in any of claims 7 to 9, or a textile impregnated with said mixture.

## Revendications

1. Composés à unités carbodiimide et à groupes carboxyle ou carboxylate (composés V), dérivés
a) de polyisocyanates en C₄-C₂₀ aliphatiques ou araliphatiques (composant a),
b) d'acides hydroxycarboxyliques ou de sels d'acide hydroxycarboxylique (composant b), et
c) éventuellement d'autres composés qui portent des groupes qui peuvent réagir avec des groupes isocyanate dans une réaction d'addition (composant c),
d) éventuellement d'autres isocyanates (composant d),
les unités carbodiimide dérivant sensiblement exclusivement des groupes isocyanate du composant (a) .

2. Composés (V) suivant la revendication 1, contenant 200 à 2000 mmoles/kg de groupes carboxyle ou carboxylate, par rapport au poids des composés.

3. Composés (V) suivant la revendication 1 ou 2, dans lesquels, pour ce qui concerne le composant (a), il s'agit de diisocyanate d'hexaméthylène ou de 1,3-bis-(1-méthyl-1-isocyanato-éthyl)benzène.

4. Composés (V) suivant les revendications 1 à 3, dans lesquels, pour ce qui concerne les acides hydroxycarboxyliques et respectivement les sels d'acide hydroxycarboxylique, il s'agit d'acides carboxyliques ou respectivement de sels comportant un ou deux groupes hydroxyle.

5. Composés (V) suivant les revendications 1 à 4, dans lesquels, pour ce qui concerne le composant (c), il s'agit de composés aromatiques, aliphatiques ou araliphatiques portant éventuellement des groupes oxyde de polyalkylène qui présentent 1 à 20 atomes de carbone (dans le nombre cité d'atomes de carbone ceux des groupes oxyde de polyalkylène ne sont pas compris) et au moins un groupe fonctionnel, choisi parmi le groupe des groupes amino secondaires, des groupes amino primaires ou des groupes hydroxyle alcoolique.

6. Procédé de préparation de composés (V) suivant les revendications 1 à 5, dans lequel
I. on prépare des carbodiimides à groupes isocyanate terminaux, en carbodiimidisant une partie des groupes isocyanate du composant (a), et
II. on fait réagir les composés préparés selon l'étape I qui comportent des groupes isocyanate terminaux avec le composant (b) et éventuellement les composants (c) et (d).

7. Mélanges d'un composé (V) suivant les revendications 1 à 5 et d'une dispersion aqueuse contenant un polymère (P).

8. Mélanges suivant la revendication 7, dans lesquels le polymère (P) porte des groupes carboxyle.

9. Mélanges suivant la revendication 7, dans lesquels, pour ce qui concerne le polymère (P), il s'agit d'un polyuréthanne (PII) constitué
IIa) de diisocyanates comportant 4 à 30 atomes de C,
IIb) de diols, dont
IIb1) 10 à 100% molaires, par rapport à la quantité globale des diols (IIb), présentent un poids moléculaire de 500 à 5000, et
IIb2) 0 à 90% molaires, par rapport à la quantité globale des diols (IIb), présentent un poids moléculaire de 60 à 500 g/mole,
IIc) de monomères différents des monomères (IIa) et (IIb) comportant au moins un groupe isocyanate ou au moins un groupe réactif vis-à-vis des groupes isocyanate, qui portent en outre au moins un groupe hydrophile ou un groupe potentiellement hydrophile, la capacité de dispersion dans l'eau des polyuréthannes étant ainsi produite,
IId) éventuellement d'autres composés polyvalents différents des monomères (IIa) à (IIc) qui présentent des groupes réactifs pour lesquels il s'agit de groupes hydroxyle alcoolique, de groupes amino primaires ou secondaires ou de groupes isocyanate, et
IIe) éventuellement de composés monovalents différents des monomères (IIa) à (IId) présentant un groupe réactif pour lequel il s'agit d'un groupe hydroxyle alcoolique, d'un groupe amino primaire ou secondaire ou d'un groupe isocyanate.

10. Mélanges suivant la revendication 7, dans lesquels, pour ce qui concerne le polymère (P), il s'agit d'un polymère (PIII), constitué
IIIa) de 30 à 99,9% en poids de monomères principaux choisis parmi des (méth)acrylates d'alkyle en C₁-C₂₀, des esters vinyliques d'acides carboxyliques contenant jusqu'à 20 atomes de C, de substances vinyl-aromatiques comportant jusqu'à 20 atomes de C, des nitriles éthyléniquement insaturés, des halogénures de vinyle et des hydrocarbures aliphatiques comportant 2 à 8 atomes de C et 1 ou 2 doubles liaisons,
IIIb) de 0 à 20% en poids d'un acide carboxylique présentant une double liaison oléfinique, et
IIIc) de 0 à 20% en poids de monomères polymérisables par voie radicalaire, différents de (IIIa) et de (IIIb).

11. Objets, qui sont collés ou revêtus d'un mélange suivant les revendications 7 à 9, ainsi que des matières textiles qui sont imprégnées par ce mélange.
